# EUROPEAN PATENT SPECIFICATION

(11) **EP 3 532 040 B1**
(45) Date of publication and mention of the grant of the patent: **29.11.2023**
(21) Application number: 17788146.3
(22) Date of filing: 09.10.2017
(51) Int. Cl.: A61K 31/12, C07C 49/573, A61Q 19/00, A61Q 11/00, A61K 8/35, C07C 45/64, C07C 49/577

(54) **A PERSONAL CARE COMPOSITION**
KÖRPERPFLEGEZUSAMMENSETZUNG
COMPOSITION DE SOINS PERSONNELS

(30) Priority: 27.10.2016 WO PCT/CN2016/103515; 02.12.2016 EP 16201871
(43) Date of publication of application: 04.09.2019
(73) Proprietor: Unilever Global IP Limited, Wirral, Merseyside CH62 4ZD (GB); Unilever IP Holdings B.V., 3013 AL Rotterdam (NL)
(72) Inventor: HARDING, Clive, Roderick, Little Neston Cheshire CH64 4AF (GB); HARICHIAN, Bijan, Trumbull, Connecticut 06611 (US); ROSA, Jose, Guillermo, Trumbull, Connecticut 06611 (US); ZHOU, Luxian, Shanghai 201114 (CN)
(74) Representative: Tansley, Sally Elizabeth
(86) International application number: PCT/EP2017/075643
(87) International publication number: WO 2018/077602

(56) References cited:
- WO-A1-2012/076696
- WO-A2-2011/082290

## Description

### Field of the Invention

The invention relates to a novel compound and to a process to prepare it. It further relates to a personal care composition that prevents or reduces inflammation. The composition could be delivered in the form of a skin, scalp, hair or oral care product.

### Background of the Invention

inflammation, a complicated biological host response to harmful stimuli, is a mechanism by which the host removes the stimuli and initiates the healing process for self-protection. The innate immune system for a host is the first line of defence against invading organisms in a non-specific manner. Dysregulated inflammation may cause various personal care problems including gingivitis/periodontitis (in the oral cavity), dandruff (on scalp/ hair) and eczema/acnes (on skin). To assist the host organism (e.g. the human or animal) several anti-inflammatory agents either through topical application or through oral consumption have been developed and used to mitigate the above problems.

Many products that we consume have a negative impact on our teeth and mouth. Acidic drinks and sweets, for example, can result in tooth erosion by attacking enamel which is the outer coating that protects the teeth. Foods which are sticky can accumulate in the interstices between teeth and in the region between the gums and the teeth. In the absence of good oral hygiene, these can harden over time with the help of oral bacteria to form a film known as plaque.

Gingivitis is an inflammatory process of the gums caused by accumulation of plaque and/or bacteria. During gingivitis, the bacteria residing in the dental plaque biofilms and its corresponding components interact with gingival tissues. Following this, innate immune response is activated, characterised by the release of pro-inflammatory cytokines. Gingivitis is a mild phase of periodontal disease and defined as reversible inflammation. It is believed that good habit of oral hygiene e.g. brushing and using mouth rinse product with therapeutic anti-microbial and anti-inflammatory efficacy can be an effective way for individuals to reduce the plaque build-up that causes gingivitis.

Chronic gingivitis results in mild bleeding from the gums during tooth brushing. Gingivitis can progress to a more severe state (chronic periodontitis) when the inflammatory process extends to the periodontal ligament and alveolar bone and/or exert a significant systemic impact on health. Chronic periodontitis is asymptomatic until teeth shift, loosen, or are lost.

Dandruff is a condition experienced by many people worldwide. The dandruff condition varies from mild symptoms such as flaking skin to severe inflammation and itchiness of the scalp. Malassezia yeasts, such as *Malassezia furfur*, are believed by some to be the main cause of dandruff and, whilst this might not represent the full scientific picture of the situation, Malassezia yeasts do appear to be closely associated with dandruff. Hence, the strategy conventionally used for the treatment of dandruff is the topical application of antifungals such as zinc pyrithione (ZnPTO), octopirox, climbazole and ketoconazole which are normally delivered through a shampoo. Additionally, anti-inflammatory agents have also been used in anti-dandruff products to alleviate the ill-effects of this condition.

On the skin, one of the problems experienced by many people, especially on the face, is acne. This has a displeasing cosmetic appearance. Acne, also known as Acne vulgaris, is a common skin condition that affects nearly all adolescents and adults at some times in their lives. It has a complex etiology, involving abnormal keratinization, excess sebum production, androgen function, bacterial growth, and immune hypersensitivity. Although one or more of the above processes is correlated with acne, the one triggering factor and the exact sequence of events leading to the formation of acne lesions has not been fully understood. Other factors which have been linked to acne are presence of free radicals with subsequent oxidative stress leading to cellular damage. It has been observed that acne usually occurs in areas rich in sebaceous glands like the face, neck and back. A bacteria *Propionibacterium acnes (P. acnes)* has also been implicated in occurrence of acne.

Acne has been treated in many ways. Most treatments take several weeks to months before a noticeable change is seen. Benzoyl peroxide which has an antibacterial effect has been used for mild cases of acne and is also believed to prevent formation of further acne. In very severe cases of acne, antibiotics like tetracycline, erythromycin and clindamycin have been used. Antibiotics are believed to work by several mechanisms, the most important being the decrease in the number of bacteria in and around the follicle. They are also thought to reduce the irritating chemicals produced by the white blood cells in the sebum, thereby reducing the inflammatory response.

Thus, inflammation is a process that is manifest on the topical surface of the human or animal body in one or all of the above described conditions. The present inventors have attempted to alleviate the symptoms of the above conditions by developing new actives as well as exploring combination of actives that exhibit synergistic anti-inflammatory benefits. The present invention concerns identification of actives which could be derivatized from compounds found in extracts of natural materials. Natural materials from which many actives have been extracted include ginger, turmeric, tea, grape, tomato and a host of others. One such active is curcumin which has been known for a long time to alleviate very many health and cosmetic problems. The present inventors have taken curcumin and tried to derivatise it to new compounds hoping to find actives that have anti-inflammatory properties. Curcumin is an active that is extractable from the natural rhizome turmeric. Turmeric has been used as a spice in cooking and has a distinctive yellow colour. It is known to have antimicrobial and anti-inflammatory properties. Curcumin has the structure as give below: 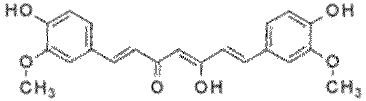 Enol form 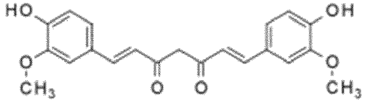 Keto form

Although curcumin has very many therapeutic properties which enable it to be used in medical and cosmetic treatments, one of the negatives is that it has a strong yellow colour which impedes the flexibility in preparing cosmetic compositions where visual appeal is very important. The present inventors have tried to retain the structural and spatial attributes of the curcumin backbone while attempting to minimize the yellowness attribute of the synthesized compound. After extensive experiments involving synthesizing hundreds of such compounds, they arrived at the present invention.

It is thus an object of the present invention to provide for a novel anti-inflammatory active that is effective for use in personal care.

It is another object of the present invention where the active can be derived from a compound extractable from a natural source like curcumin.

It is yet another object of the present invention to prepare an active which is a derivative of curcumin which has significantly lower yellow colour.

### Summary of the Invention

According to the first aspect of the present invention there is provided a compound having the chemical structure of formula 1 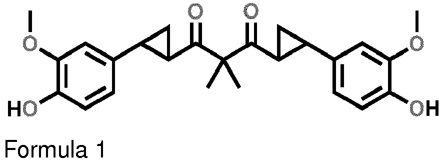 1,3-bis(2-(4-hydroxy-3-methoxyphenyl)cyclopropyl)-2,2-dimethylpropane-1,3-dione.

According to another aspect of the present invention there is provided a process to prepare a compound of the first aspect comprising the steps of:
(i) protection of the phenolic hydroxyl groups of curcumin with a suitable protecting group P to generate a compound with formula 2
(ii) di-methylation of compound with formula 2 to generate a compound with formula 3
(iii) Di-cyclopropanation of compound with formula 3 to generate a compound with formula 4
(iv) deprotection of the phenolic hydroxyl groups of compound with formula 4 to generate compound with formula 1.

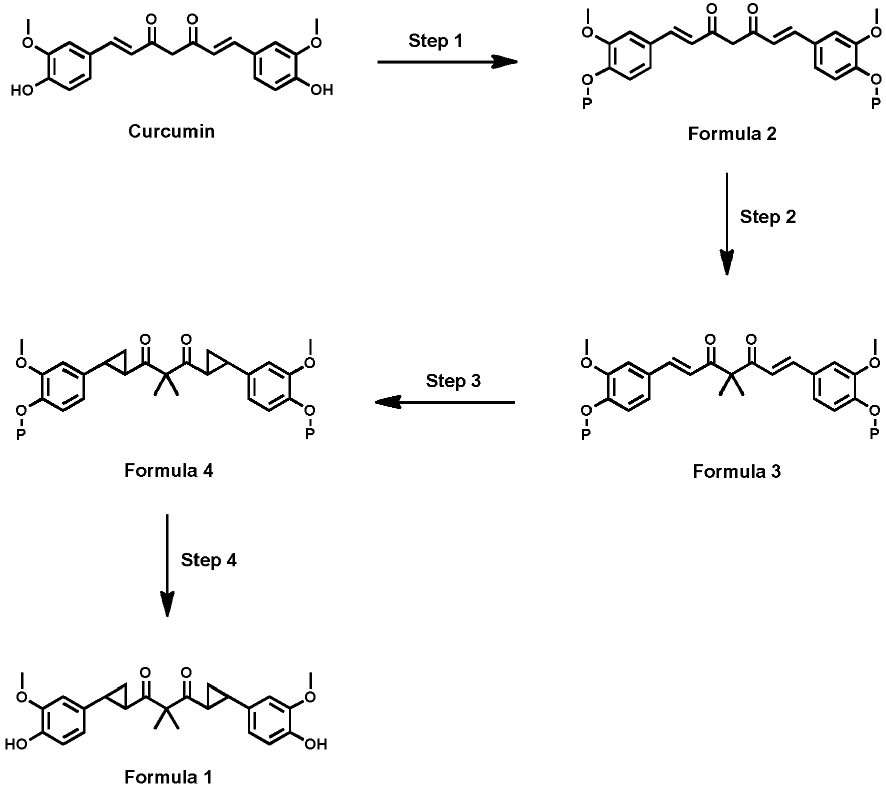

According to yet another aspect of the present invention there is provided a personal care composition comprising the compound of the first aspect and a cosmetically acceptable base.

According to yet another aspect of the present invention there is provided a compound of the first aspect for use in reducing inflammation on a topical surface of a human or animal body comprising the step of applying the compound on to the desired surface.

### Detailed description of the Invention

Except in the examples, or where otherwise explicitly indicated, all numbers in this description indicating amounts of material or conditions of reaction, physical properties of materials and/or use may optionally be understood as modified by the word "about". All amounts are by weight of the final composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

"Oral care composition" for the purposes of the present invention means a paste, powder, liquid, gum, serum or other preparation for cleaning the teeth or other surfaces in the oral cavity.

"Tooth paste" for the purpose of the present invention means a paste or gel dentifrice for use with a toothbrush. Especially preferred are tooth pastes suitable for cleaning teeth by brushing for about two minutes.

"Mouth wash" for the purpose of the present invention means liquid dentifrice for use in rinsing the mouth. Especially preferred are mouth washes suitable for rinsing the mouth by swishing and/or gargling for about half a minute before expectorating.

Viscosity of a composition is the value taken at room temperature (25°C) with a Brookfield Viscometer, Spindle No.4 and at a speed of 5 rpm. Values are quoted in centipoises (cP=mPa.s) unless otherwise specified.

By a 'Hair Care Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and wash-off shampoos, conditioners, shower gels, or toilet bar. The composition of the present invention is preferably a wash-off composition, especially preferred being a shampoo or a conditioner and most preferably a shampoo.

"Water-insoluble", as used herein, refers to the solubility of a material in water at 25°C and atmospheric pressure being 0.1% by weight or less.

By 'A topical composition' or a 'skin care composition' as used herein, is meant to include a composition for topical application to the skin of mammals, especially humans. Such a composition could be of the leave-on or of the wash-off/ rinse-off type. By a leave-on composition is meant a composition that is applied to the desired skin surface and left on for a period of time (say from one minute to 24 hours) after which it may be wiped or rinsed off with water, usually during the regular course of personal washing. By a wash-off/ rinse off composition is meant a composition that is applied to the desired skin surface for a shorter period of time say of the order of seconds or minutes and usually contains sufficient surfactants that aids in cleaning the surface which may be rinsed off with copious amounts of water. The composition may also be formulated into a product which is applied to a human body for improving the appearance, cleansing, odor control or general aesthetics. The composition of the present invention can be in the form of a liquid, lotion, cream, foam, scrub, gel or stick form and may be delivered through a roll-on device or using a propellant containing aerosol can. "Skin" as used herein is meant to include skin on any part of the body e.g., neck, chest, back, arms, underarms, hands, legs, buttocks and scalp. When the product is used for the underarms it is usually called a deodourant procuct or a deo product. A class of deodourant product is the so called anti-perspirant (AP) product which contains an AP active which when applied to the axilla of an individual delivers anti-perspirancy and deodourancy benefits.

It is desirable that the color of the novel compound of the present invention lies within the colorless to light yellow range for optimum visual appeal and usage in topical and/or skin care compositions. Visual assessment of color from a solid or solution sample of novel compounds in comparison to a solid or solution sample of curcumin provides a means to determine relative reduction of color and appeal potential. In addition, UV-Vis spectra of solution samples of novel compounds in comparison to a solution sample of curcumin provides a means to measure color experimentally and allows a more direct measurement of color. Thus, using these two simple measurements it is possible to assess the relative reduction in color of novel inventive compounds relative to curcumin and allows prioritization of compounds for further development.

The present invention relates to the novel compound of formula 1 given below: 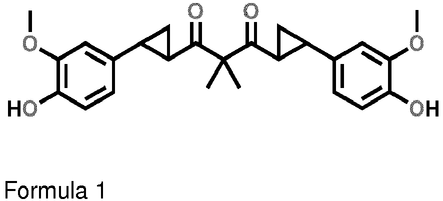 1,3-bis(2-(4-hydroxy-3-methoxyphenyl)cyclopropyl)-2,2-dimethylpropane-1,3-dione.

Compound of formula 1 has several unique and desirable properties over curcumin, including significantly improved color (light yellow for compound of formula 1 versus orange for curcumin), significantly improved solubility in a broad spectrum of solvents and reduced melting point temperature [gel for compound of formula 1 versus high melting solid (183 °C) for curcumin].

The various chemical process conditions for each of Steps 1 to 4 under which this novel compound may be synthesized are given below:

In Step 1, curcumin is dissolved in an organic solvent system selected from but not limited to dichloromethane, chloroform, 1,2-dichloroethane, ethyl acetate, isopropyl acetate, isobutyl acetate, acetonitrile, tetrahydrofuran, 2-methyltetrahydrofuran, diethoxymethane, methyl t-butyl ether and mixtures thereof. The concentration of curcumin in the organic solvent may range between 0.001M to 10M, more preferably in the range between 0.01M to 1M. A protecting group reagent is added such that when it reacts with the phenolic hydroxyl groups of curcumin it results in the formation of compound with formula 2 where P is the structure of a protecting group covalently attached to the phenolic oxygen atom and further blocks or "protects" the phenolic hydroxyl groups from further reactions during Steps 2 and 3 of the chemical process leading to compound with formula 1. Suitable protecting groups P used in Step 1 include but are not limited to tetrahydropyranyl, 4-methoxytetrahydropyranyl, tetrahydrofuranyl, methoxymethyl, 1-ethoxyethyl, 1-methyl-1-methoxyethyl, t-butyl, triphenylmethyl, alpha-naphthyldiphenylmethyl, p-methoxyphenyldiphenylmethyl, isopropyldimethylsilyl, t-butyldimethylsilyl, tribenzylsilyl or triisopropylsilyl. The amount of protecting group reagent added can range from 2-50 equivalents with respect to the number of curcumin equivalents, more preferably between 2 to 20 equivalents. A suitable catalyst (e.g. acidic such as p-toluenesulfonic acid or weakly basic such as imidazole) selected based on the protecting group reagent used is added in the range between 0.01 to 1 equivalents with respect to the number of curcumin equivalents. The reaction may be performed between 10 to 50 °C, more preferably between 15 to 35 °C but not limited to these temperatures. The progress of the reaction is monitored by a suitable detection method such as UV-Vis visualization, thin layer chromatography and/or high performance liquid chromatography. At the point where no further formation of product is detected, the reaction is processed to allow product isolation. Typically, processing of the reaction mixture to isolate compound of formula 2 involves but is not limited to removal of the reaction solvent under reduced pressure between 20 to 5 °C, followed by an extraction process (e.g. dissolving the crude reaction mixture in an organic solvent and washing with aqueous buffers to remove impurities) to isolate the crude product, and final purification of the crude product using flash chromatography on silica gel or crystallization using a solvent or mixed solvent system to give pure compound with formula 2.

In Step 2, compound with formula 2 is dissolved in an organic solvent system selected from but not limited to dimethylsulfoxide, N,N-dimethylformamide, N-methylpyrrolidone, methanol, ethanol, isopropanol, t-butanol, t-amyl alcohol, acetone, 2-butanone, chloroform, 1 ,2-dichloroethane, ethyl acetate, isopropyl acetate, isobutyl acetate, acetonitrile, tetrahydrofuran, 2-methyltetrahydrofuran, diethoxymethane, methyl t-butyl ether, water, glycerol, 1 ,2-propanediol and mixtures thereof to generate a biphasic or monophasic system. The concentration of compound with formula 2 in the solvent system may range between 0.001M to 10M, more preferable in the range between 0.01M-1M. A base selected but not limited to an alkali or alkaline earth bicarbonate, carbonate or hydroxide is added, followed by a methylating agent selected from but not limited to iodomethane, bromomethane, chloromethane, dimethylcarbonate, dimethyldicarbonate, dimethylsulfate, methylfluorosulfonate, methyl methanesulfonate, methyl trifluoromethanesulfonate and trimethyloxonium tetrafluoroborate. Both, the amount of base reagent and methylating agent added can range from 2 to 10 equivalents with respect to the number of formula 2 equivalents, more preferably between 2 to 5 equivalents. For biphasic solvent systems a catalyst selected but not limited to quaternary ammonium salts or organic phosphonium salts is added in the range between 0.001 to 1 equivalents with respect to the number of formula 2 equivalents. The reaction can be performed between 10 to 50 °C, more preferably between 15 to 35 °C but not limited to these temperatures. The progress of the reaction is monitored by a suitable detection method such as UV-Vis visualization, thin layer chromatography and/or high performance liquid chromatography. At the point where no further formation of product is detected the reaction is processed to allow product isolation. In the case of monophasic reaction solvent systems, processing of the reaction mixture to isolate compound of formula 3 involves an extraction process (e.g. dissolving the crude reaction mixture in an organic solvent and washing with aqueous buffers to remove impurities) to isolate the crude product, removal of solvents under reduced pressure between 30 to 65 °C, and final purification of the crude product using flash chromatography on silica gel or crystallization using a solvent or mixed solvent system to give pure compound with formula 3. In the case of biphasic reaction solvent systems, the organic layer is separated from the aqueous layer and the solvents removed under reduced pressure between 20 to 65 °C, followed by an extraction process and purification as above.

In Step 3, compound with formula 3 is dissolved in an organic solvent system selected from but not limited to dimethylsulfoxide, N,N-dimethylformamide, N-methylpyrrolidone, acetonitrile, tetrahydrofuran, 2-methyltetrahydrofuran or an ionic liquid (e.g. 1-butyl-3-methylimidazolium hexafluorophosphate) and mixtures thereof and the solution added to a pre-mixed mixture of trimethylsulfoxonium iodide with a base selected but not limited to an alkali or alkaline earth hydride or hydroxide or tertiary amine base in any of the solvent systems above. The concentration of compound with formula 3, trimethylsulfoxonium iodide and base in the solvent system may range between 0.001M to 5M, more preferable in the range between 0.01M to 1M. Both, the amount of trimethylsulfoxonium iodide and base reagent can range from 2 to 5 equivalents with respect to the number of formula 3 equivalents, more preferably between 2 to 3 equivalents. The reaction can be performed between 0 to 50 °C, more preferably between 10 to 35 °C but not limited to these temperatures. The progress of the reaction is monitored by a suitable detection method such as UV-Vis visualization, thin layer chromatography and/or high performance liquid chromatography. At the point where no further formation of product is detected the reaction is processed to allow product isolation. Typically, processing of the reaction mixture to isolate compound of formula 4 involves an extraction process (e.g. dissolving the crude reaction mixture in an organic solvent and washing with aqueous buffers to remove impurities) to isolate the crude product, removal of solvents under reduced pressure between 30 to 65 °C, and final purification of the crude product using flash chromatography on silica gel or crystallization using a solvent or mixed solvent system to give pure compound with formula 4.

In Step 4, removal of the protecting group P is achieved by dissolving compound with formula 4 in an organic solvent system appropriate and compatible with the conditions needed to remove protecting group P to generate compound with formula 1. A non-exclusive list of solvents applicable in this case include but are not limited to chlorinated solvents (e.g. dichloromethane), alcohols (e.g. ethanol), diols (e.g. 1,2-propanediol), triols (e.g. glycerol), ethers (e.g. tetrahydrofuran, t-butyl methyl ether), water, esters (e.g. ethyl acetate), dimethylsulfoxide, dimethylformamide, acetonitrile and mixtures thereof. A suitable deprotection reagent (e.g. acidic such as acetic acid or weakly basic such as sodium fluoride) depending on the nature of the protecting group P to be removed is added in the range between 0.01 to 5 equivalents with respect to the number of curcumin equivalents. The reaction can be performed between 0 to 50 °C, more preferably between 15 to 35 °C but not limited to these temperatures. The progress of the reaction is monitored by a suitable detection method such as UV-Vis visualization, thin layer chromatography and/or high performance liquid chromatography. At the point where no further formation of product is detected the reaction is processed to allow product isolation. Typically, processing of the reaction mixture to isolate compound of formula 1 involves but is not limited to removal of the reaction solvent under reduced pressure between 20 to 55 °C, followed by an extraction process (e.g. dissolving the crude reaction mixture in an organic solvent and washing with aqueous buffers to remove impurities) to isolate the crude product, and final purification of the crude product using flash chromatography on silica gel or crystallization using a solvent or mixed solvent system to give pure compound with formula 1.

Once this compound is prepared, it is generally included at 0.01 to 5%, preferably at 0.1 to 3%, more preferably at 0.1 to 2% by weight of the personal care composition.

The composition of the invention may be prepared so that it is suitable for use as an oral care or a skin, scalp or hair care product. The product may be delivered in the form of a solid, soft solid, liquid, emulsion, microemulsion, lotion, cream, gel, or aerosol forms.

### Oral Care

When the personal care composition is delivered for oral care, it includes a cosmetically acceptable base which may be an abrasive, a thickener, a humectant or an orally acceptable surfactant. The product may be delivered in the form of an ointment, a gel, a dentifrice or a mouthwash.

Oral care compositions preferably comprise an abrasive. Gels usually contain silica, whereas opaque creams generally contain calcium based abrasives, especially chalk.

Preferred toothpaste compositions have 5 to 60 wt% calcium based abrasive. In more preferred compositions it is 30 to 60 wt% and further more preferably from 35 to 55 wt%. Optimal compositions have 40 to 55wt% calcium based abrasive.

A preferred abrasive is fine ground natural chalk (FGNC), which is a form of chalk. It is obtained from limestone or marble. FGNC may also be modified chemically or physically by coating during milling or after milling by heat treatment. Typical coating materials include magnesium stearate or oleate. The morphology of FGNC may also be modified during the milling process by using different milling techniques, for example, ball milling, air-classifier milling or spiral jet milling.

FGNC may be used as the sole calcium based abrasive. However, FGNC may also be used with the other calcium based abrasives for some balance of abrasion. Usually the particle size of chalk is from 1 to 60 µm, and preferred sizes range from 1 to 15 µm. Other preferred calcium based abrasives include dicalcium phosphate (DCP), calcium pyrophosphate and precipitated calcium carbonate (PCC), which preferably are included at 25 to 55 wt%, more preferably 35 to 50 wt%.

When a combination of calcium based abrasives is desired, it is preferred that FGNC is 35 to 100%, more preferably 75 to 100% and especially from 95 to 100% of the total amount of Calcium based abrasives. In such cases, the balance, most preferably, is PCC.

Other abrasives may also be used depending upon the intended degree of abrasion. These include synthetic abrasive polishing agents such as amorphous precipitated silica and silica gels. Other abrasive agents include magnesium carbonate, sodium metaphosphate, potassium metaphosphate, zirconium silicate, potassium metaphosphate, magnesium orthophosphate, tricalcium phosphate, magnesium orthophosphate, trimagnesium phosphate, aluminum silicate, zirconium silicate and perlite.

In a preferred embodiment, the composition comprises a thickener. Thickeners that may be used in this invention include, sodium carboxymethyl cellulose (SCMC), hydroxyl ethyl cellulose, methyl cellulose, ethyl cellulose, gum tragacanth, gum arabic, gum karaya, xanthan gum, sodium alginate, carrageenan gum, guar gum, Irish moss, starch, modified starch, silica based thickeners including silica aerogels, magnesium aluminum silicate (e.g., Veegum^{®}), Carbomers (cross-linked acrylates) and mixtures thereof.

Typically, thickening silica, sodium carboxymethyl cellulose and/or a Carbomer is/are preferred thickeners for use in the composition of the invention. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen^{®} PNC, Synthalen^{®} KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickening silica is especially preferred to be used in gel toothpastes.

Gel toothpastes generally contain upto 8.5 wt% thickening silica whereas opaque toothpastes typically contain 3 to 4 wt% thickening silica.

When present, preferred thickening silicas include AEROSIL T series from Degussa or the CAB-O-SIL series from Cabot Corporation, silica gels such as the SYLODENT or SYLOX series from W. R.Grace & Co or precipitated silica such as ZEOTHIX 265 from J. M. Huber Corporation. Useful silica thickeners also include ZEODENT 165, ZEODENT 163 and/or 167 and ZEOFREE 153, 177, and/or 265 silicas, all available from J. M. Huber Corporation. Other preferred thickening silicas include MFIL, MFIL-P (From Madhu Silica, India), SIDENT 22 S and AEROSIL 200 (Ex. Evonik Industries), SYLODENTand PERKASIL thickening silicas from WR Grace & Company and Tixosil 43 and 331 from Rhodia, synthetic finely divided pyrogenic silica such as those sold under the trademarks SYLOID 244, SYLOID 266 and AEROSIL D-200.

Thickener, when present, preferably makes up from 0.01 to about 10%, more preferably from 0.1 to 9%, and most preferably, from 1.5 to 8% by weight of the composition.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of oral care compositions. More preferably, the humectant makes up from 25 to 80%, and most preferably, from 45 to 70% by weight of the composition, based on total weight of the composition and including all ranges subsumed therein.

Preferably, an oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. More preferably the surfactant comprises or is an anionic surfactant. The preferred anionic surfactants are sodium lauryl sulphate and/or sodium dodecylbenzene sulfonate. Most preferably the surfactant is sodium lauryl sulphate. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. Most preferred surfactants are an alkali metal alkyl sulphate or a betaine.

Water may preferably be included in 5 to 95%, in particular 10 to 75%, and especially at from 10 to 60%, further more preferably 10 to 45% by total weight of the composition.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

The oral care composition of the present invention may contain a variety of other ingredients which are common in the art to enhance physical properties and performance. These ingredients include antimicrobial, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

### Hair Care

As per a preferred aspect of the invention, the composition may be used for hair care. It is especially useful for preventing or alleviating the symptoms of dandruff. One medium through which this may be delivered is that of a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, further more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES).

Preferred ethoxylated alkyl sulfate anionic surfactant is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

Hair care compositions whether delivered as shampoos or conditioners usually comprise an anti-dandruff agent. The most preferred anti-dandruff agent for use in the compositon of the invention is a zinc based anti-dandruff agent preferably zinc pyrthione. Zinc pyrithione belongs to the class of insoluble metal pyrithione which may be represented by the following general formula: 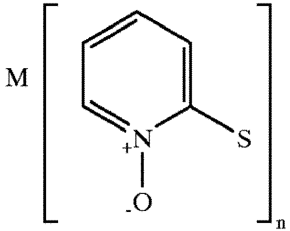 in which M is a polyvalent metal ion and n corresponds to the valency of M. In the present invention M corresponds to Zinc and n has the value of 2.

The zinc pyrithione may have any particle form suitable for use in a composition for topical application. For example, the zinc pyrithione may be in the form of amorphous or crystalline particles having a range of different particle sizes. The zinc pyrithione may, for example, be in the form of particles having a size distribution in which at least about 90 % of the particles have a size of up to 100 microns, more preferably up to 50 microns, even more preferably up to 10 microns, most preferably 5 microns or less.

Various methods for producing fine particles of metal pyrithione are described, for example, in EP-A-0 173 259. Suitable methods for determining particle size are described in that document. The insoluble metal pyrithione may be made up of one particulate form or two or more different particulate forms.

Other suitable particulate forms for the zinc pyrithione include platelets and needle-shaped particles. Platelets of zinc pyrithione are described in EP0034385. The needleshaped particles are preferably of the type described in WO99/66886. For needle-shaped particles preferably at least 50% by number of the particles are needle-shaped particles having a length of between 1 µm and 50 µm.

The amount of metal pyrithione incorporated into the compositions may depend on the type of composition and the exact nature of the material used. A preferred amount of pyrithione is from about 0.01% to about 1.5% by weight of the total composition, more preferably from about 0.05% to about 1.5% by weight of the total composition.

The composition as per the invention especially for anti-dandruff shampoos preferably additionally comprises a zinc compound. The presence of additional zinc compound in the composition is believed to improve the antidandruff efficacy of the zinc based antidandruff agent. Suitable zinc compounds are zinc oxide, zinc citrate, zinc malonate, zinc carbonate or combinations thereof. The zinc compound is preferably present in 0.1 to 3%, more preferably 0.1 to 1.5% by weight of the composition.

Shampoo composition as per the invention preferably additionally comprises a conazole fungicide. Preferably the conazole fungicide is selected form ketoconazole, climbazole or mixtures thereof. The azole fungicide is preferably included in 0.01 to 2%, more preferably 0.025 to 0.75% by weight of the composition. The presence of a conazole fungicide is believed to improve the deposition of zinc pyrithione.

### Skin Care

The composition of the invention may be used for skin care. The cosmetically acceptable base in such cases may be a liquid or solid material. Typically, base is present in an amount ranging from 10 to 99.9%, more preferably from 20 to 95%, most preferably from 40 to 85% by total weight of the composition including all ranges subsumed therein. It is particularly preferred that the cosmetically acceptable carrier includes water. Water is preferably included in an amount from 30 to 90%, more preferably from 30 to 85%, most preferably from 30 to 80% by total weight of the sunscreen composition. Besides water, suitable carrier classes include silicones, polyhydric alcohols, hydrocarbons, triglycerides and thickening powders.

The skin care composition of the invention may be in any form including toners, lotions, creams, mousses, scrub, serum or gel that is suitable for topical application to the skin. The composition can be either a leave-on product such as skin lotions, creams, antiperspirants, deodorants, lipsticks, foundations, mascara, sunless tanners and sunscreen lotions or a rinse-off product such as shower gels and toilet bars. It is preferred that the composition is a skin lotion or a cream.

The composition may comprise an emollient oil that act as a co-solvent. Suitable emollient oils include, for example, ester of alkoxylated aromatic alcohol with fatty carboxylic acid, esters of polyglycols or diols with fatty carboxylic acid such as caprylic/capric triglyceride, ester of fatty alcohol and fatty acid, alkoxylated derivative of benzyl alcohol and mixtures thereof. Preferably the emollient oil is caprylic/capric triglyceride.

Typically, such compositions comprise co-solvent in an amount from 0.01 to 10%, more preferably from 0.1 to 8%, most preferably from 1 to 6%, based on the total weight of the sunscreen composition and including all ranges subsumed therein.

The composition may additionally comprise sunscreen agents such as inorganic sunscreens. For example, zinc oxide, titanium dioxide, iron oxide, silica such as fumed silica. The amount of such sunscreen agents is preferably incorporated from 0.1 to 5% by total weight of the sunscreen composition.

The composition of the invention may comprise a UV-A sunscreen agent selected from the group consisting of a dibenzoylmethane derivative, a triazine derivative, a benzophenone derivative and mixtures thereof. In a preferred embodiment, the UV-A sunscreen agent comprises or is a dibenzoylmethane derivative, for example, butyl methoxydibenzoylmethane (sold under the trade name Parsol 1789).

Typically, the sunscreen composition of the present invention comprises from 0.1 to 15% by weight of the UV-A sunscreen agent, more preferably from 0.1 to 10%, most preferably from 1 to 5%, based on the total weight of the composition and including all ranges subsumed therein.

The composition of the invention may also comprise a UV-B sunscreen agent. Suitable UV-B sunscreen agent of the invention is selected from the group consisting of a benzophenone, an anthranilate, a salicylate, a cinnamate, a camphor, benzylidene malonate, a triazone, and derivatives thereof. In a preferred embodiment, the UV-B sunscreen agent comprises or is a cinnamate derivative, for example, ethylhexyl methoxycinnamate (sold under the trade name Parsol MCX).

Typically, the composition comprises from 0.1 to 20% by weight of the UV-B sunscreen agent, more preferably from 0.5 to 18%, most preferably from 1 to 15%, based on the total weight of the composition and including all ranges subsumed therein.

A skin lightening agent may also be incorporated into the composition of the invention. Most preferred skin lightening active is a Vitamin B3 compound. Vitamin B3 compound maybe nicacin, nicotinic acid or niacinamide, preferably niacinamide. Niacinamide has the structure given below:

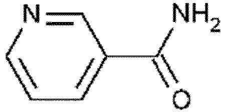

Niacinamide is preferably present in 0.01 to 5%, more preferably 0.1 to 3% by weight of the composition. Suitable skin lightening agents other than Vitamin B3 and its derivatives are kojic acid, arbutin, tranexamic acid, placental extract, ascorbic acid and its derivatives (e.g. magnesium ascorbyl phosphate, sodium ascorbyl phosphate, ascorbyl glucoside, and ascorbyl tetraisopalmitates), aloe extract, ammonium lactate, azelaic acid, citrate esters, ellagic acid, glycolic acid, green tea extract, hydroquinone, lemon extract, linoleic acid, vitamins like vitamin B6, vitamin B12, vitamin C, vitamin A, a dicarboxylic acid, resorcinol derivatives, hydroxycarboxylic acid like lactic acid and their salts (e.g. sodium lactate) or a mixture thereof. Typically, the skin lightening agent is present in an amount from 0.1 to 10%, more preferably from 0.2 to 5%, most preferably from 0.3 to 3% by total weight of the composition including all ranges subsumed therein.

A specific class of skin care compositions is what is known as deodourant compositions. These can be applied cosmetically and topically to the skin, broadly speaking, by one of two methods. Different consumers prefer one method or the other. In one method, sometimes called a contact method, a composition is wiped across the surface of the skin, depositing a fraction of the composition as it passes. In the second method, sometimes called the non-contact method, the composition is sprayed from a dispenser held proximate to the skin, often in the region of 10 to 20 cms. The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

There are broadly speaking two classes of contact compositions, one of which is liquid and usually applied using a roll-on dispenser or possibly absorbed into or onto a wipe, and in the second of which the desired active is distributed within a carrier liquid that forms a continuous phase that has been gelled. In one variation, the carrier fluid comprises a solvent for the desired active and in a second variation, the active remains as a particulate solid that is suspended in an oil, usually a blend of oils.

### Stick or soft solid compositions

Many different materials have been proposed as gellant for a continuous oil phase, including waxes, small molecule gelling agents and polymers, They each have their advantages and of them, one of the most popular class of gellant has comprised waxes, partly at least due to their ready availability and ease of processing, including in particular linear fatty alcohol wax gellants. A gelled deodourant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

### Roll-on

Liquid compositions that are applicable from a roll-on broadly speaking can be divided into two classes, namely those in which an active is suspended in a hydrophobic carrier, such as a volatile silicone and those in which the active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the active. Herein, such compositions will be called emulsions. Roll-on emulsions commonly comprise one or more emulsifiers to maintain a distribution of the water-soluble ingredients.

### Aerosol compositions

Deodourant compositions may be delivered through an aerosol which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally accord with one of three classes; i) low boiling point gasses liquifided by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutene. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

Skin care compositions may also comprise other ingredients which are common in the art to enhance physical properties and performance. Suitable ingredients include but are not limited to humectants, thickeners, opacifiers, binders, colorants and pigments, pH adjusting agents, preservatives, optics, perfumes, viscosity modifiers, biological additives, buffering agents, conditioners, natural extracts, essential oils and skin benefit agents including anti-inflammatory agents, cooling agents, antiperspirant agents, anti-aging agents, anti-acne agents, anti-microbial agents and antioxidants.

The invention provides for a method of reducing inflammation on a topical surface of a human or animal body comprising the step of applying the composition of the invention on to the desired surface. In one aspect the method is non-therapeutic in nature. Preferably it is for cosmetic purpose. The method may comprise applying the composition as a leave-on composition. Leave-on compositions are applied on to the desired skin surface and left thereon till the person washes the skin during the normal course of personal washing e.g. when showering or during a bath. Alternately the composition may a wash-off composition where it is used to clean the body surface and this type of composition includes a soap bar, a body wash or face wash composition or shampoo or hair conditioning composition. Alternatively, the method is for therapeutic purpose.

According to yet another aspect of the present invention there is provided use of compound of formula 1 to reduce or prevent inflammation. In one aspect the use is non-therapeutic in nature, preferably cosmetic in nature. Alternatively, it is therapeutic in nature.

According to a further aspect is disclosed a compound according to the invention for use to reduce inflammation on a topical surface of a human or animal body.

According to one more aspect is disclosed a personal care composition according to the invention for use to reduce inflammation on a topical surface of a human or animal body.

In accordance with yet another aspect is disclosed the use of a compound according to the invention in the manufacture of a composition to reduce inflammation on a topical surface of a human or animal body.

The invention will now be illustrated with the help of the following non-limiting example.

### Examples

### Examples 1-3: Anti-inflammation efficacy of compound of formula 1 in THP-1 invitro assay (using THP-1 assay)

The following procedure was used to test the anti-inflammation efficacy of the active.

THP1-XBlue^{™} (Cat No: thpx-sp, InvivoGen) cells were cultured as suspense in RPMI 1640 medium supplemented with 10% FBS, penicillin (10 U/mL) - streptomycin (10 µg/ML). Cells were differentiated in 24-well plates at the density of 5 × 10⁵ cells / well with 100 nM PMA for 72 h. Cells were then co-treated with pure *E. coli* lipopolysaccharides (LPS) and with active. After 24 h, the supernatants were collected and measured for interleukin (IL)-6 as pro-inflammatory bio-marker using enzyme-linked immunosorbent assay (ELISA).

The results in terms of concentration of IL-6 in pg/ml is given in Table -1 below:

**Table 1**

| **Examples** | **Composition** | **Concentration of IL-6 (pg/ml)** | **Std. dev** |
|---|---|---|---|
| 1 | LPS | 10508 | 485 |
| 2 | 10 µM of compound of formula 1 | 5591 | 148 |
| 3 | 20 µM of compound of formula 1 | 2996 | 144 |

### Examples 4-5: Anti-inflammation efficacy of compound of formula 1 using Human Gingival Fibroblasts assay

Primary human gingival fibroblasts (HGFs, Cat No. 2620, ScienCell^{™}) were cultured in complete fibroblast medium which included 500 mL of basal medium, 10 mL of fetal bovine serum (FBS), 5 mL of fibroblast growth supplement, and 5 mL of penicillin/streptomycin solution (Cat No. 2301, ScienCell^{™}). HGFs were seeded in 24 well-plates at the density of 2 × 10⁴ cells / well and cultured for 72 h. Cells were preincubated with active for 2 h. After removal of the active medium, the cells were washed with PBS and further treated with heat-inactivated *P. gingivalis* (Cat. 33277, ATCC). After 4 h, the supernatants were collected and measured for IL-6, IL-8 and monocyte chemoattractant protein (MCP) -1 as pro-inflammatory bio-markers using ELISA.

The data is summarised in Table - 2 below:

**Table 2**

| | | **Concentration** | | | | | |
|---|---|---|---|---|---|---|---|
| **Examples** | **Composition** | **IL-6 (pg/ml)** | **Std. dev** | **IL-8 (pg/ml)** | **Std. dev** | **MCP-1 (pg/ml)** | **Std. dev** |
| 5 | Heat activated P. gingivalis | 595 | 25 | 17100 | 2299 | 388 | 66 |
| 6 | 20 µM of compound of formula 1 | 286 | 45 | 8321 | 1938 | 232 | 21 |

The data in Table 1 and 2 indicate the anti-inflammatory efficacy of compound of formula 1 which is applicable over a wide range of personal care products.

Example 7: Synthesis of compound of formula 1: Compound of formula 1 used in the experiments in Table 1 and 2 above were synthesized using the following materials, methods and procedures:
All reagents and solvents were obtained from commercial sources (Sigma-Aldrich, EMD Chemicals) and used without further purification unless otherwise indicated.

Reaction monitoring was performed using thin layer chromatography (TLC). TLC was performed using silica gel 60 F254 plates (EMD Chemicals) and visualizing by UV (254 nm) and 4% phosphomolybdic acid (PMA) in ethanol (EtOH). Flash chromatography (FC) was performed using a Biotage SP4 system (Biotage LLC, Charlottesville, VA).

High performance liquid chromatography (HPLC) was performed using a Waters 2695 Separations Module equipped with a Waters 2996 Photodiode Array Detector and operated with Empower Pro software (Waters Corp.). Separations were carried out at 1ml/min on a Restek Pinnacle DB C18 column (5um, 4.6 X 150mm) maintained at 30 ºC. Samples for HPLC were prepared by dissolving sample in mobile phase A:B (1:1) or acetonitrile (ACN) (1mg/ml) and injecting 5-10µL onto the column. The mobile phase consisted of A = 0.1% trifluoroacetic acid (TFA) in water and B = 0.1% TFA in acetonitrile operated using gradient elution from 95:5 A:B to 5:95 A:B (gradient, 25min) followed by 100% B (isocratic, 5min). Liquid chromatography/mass spectrometry (LC-MS) was performed using a Finnigan Mat LCQ Mass Spectrometer via direct infusion of samples (50ppm) in methanol and the total ion count monitored using electrospray ionization in the (+) or (-) mode (ESI+) or (ESI-). Proton nuclear magnetic resonance (1HNMR) spectroscopy at 60 MHz was performed using an Eft-60 NMR Spectrometer (Anasazi instruments, Inc.) and processed using WinNuts^{®} software (Acorn NMR, Inc.). In selected cases 1HNMR was recorded using a 400 MHz instrument. Melting points were determined using a Meltemp^{®} apparatus (Laboratory Devices). Purity was determined by HPLC-UV/Vis. All compounds were unequivocally confirmed by LC-MS and/or 1H NMR.

The reaction scheme used to prepare a compound represented by formula 1 is shown below:

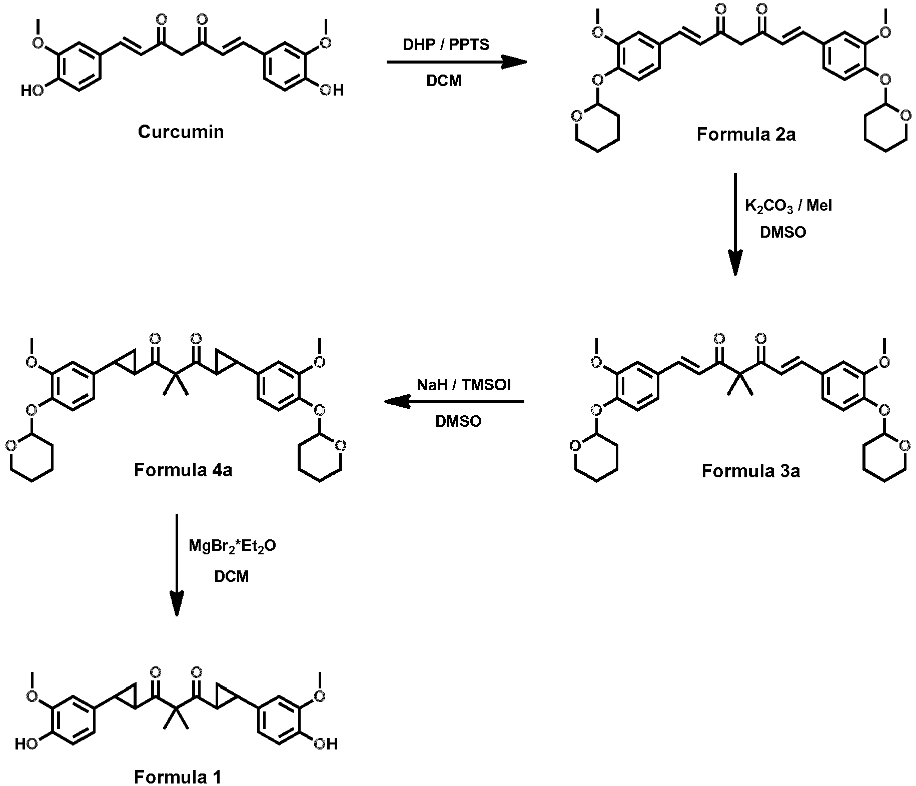

The following synthetic procedure (Steps 1 to 4) was used to prepare compound with formula 1:

### Step 1: Protection of the phenolic hydroxyl groups of curcumin to generate compound with formula 2a [(1E,6E)-1,7-bis(3-methoxy-4-((tetrahydro-2H-pyran-2-yl)oxy)phenyl)hepta-1,6-diene-3,5-dione]

Pyridinium p-toluenesulfonate (PPTS) (512mg, 2.03mmol) was added to a solution of curcumin (5g, 13.6mmol) and dihydropyran (DHP) (37.2ml, 407mmol) in anhydrous dichloromethane (DCM) (300ml) and the solution stirred at RT overnight for 16h. At this time, thin layer chromatography (TLC) [(30uL aliquot into saturated sodium carbonate (NaHCO₃):methyl t-butyl ether (MTBE) (400uL:400uL); eluting with ethyl acetate:hexane (EA:hex) (25:75)] showed the clean formation of a major product and other minor products. The reaction mixture was washed with pH 5 buffer (300ml), dried with sodium sulfate (Na₂SO₄), filtered and the solvents removed under reduced pressure to give an orange oil (9.3g). The crude product was purified by flash chromatography (FC) on silica gel eluting with EA:hex (30:70) to give pure product formula 2a as a yellow oil (4.73g, 65%). ¹H NMR (60 MHz, CDCl₃) δ 7.64 (2H, d), 7.03-7.35 (6H, m), 6.51 (2H, d), 5.83 (1H, s), 5.48-5.54 (2H, m), 3.62-4.30 (4H, m), 3.92 (6H, s), 1.27-2.10 (12H, m).

### Step 2: Di-methylation of compound with formula 2a to generate compound with formula 3a [(1E,6E)-1,7-bis(3-methoxy-4-((tetrahydro-2H-pyran-2-yl)oxy)phenyl)-4,4-dimethylhepta-1,6-diene-3,5-dione]

Potassium carbonate (K₂CO₃) (3.86g, 27.9mmol) was added to a solution of (1E,6E)-1,7-bis(3-methoxy-4-((tetrahydro-2H-pyran-2-yl)oxy)phenyl)hepta-1,6-diene-3,5-dione (3g, 5.59mmol) in anhydrous dimethylsulfoxide (DMSO) (9ml), followed by iodomethane (Mel) (1.74ml, 27.9mmol) and the mixture stirred at RT for 24h. At this time, TLC [(30uL aliquot into pH 5 buffer:EA (400uL:400uL); eluting with EA:hex (30:70)] showed the formation of a major product. The mixture was stirred for an additional 24hr and partitioned between EA (100ml) and saturated sodium chloride (NaCl) (100ml). The organic layer was dried with Na₂SO₄, filtered and the solvents removed under reduced pressure to give an orange oil (3.12g). The crude product was purified by FC on silica gel eluting with EA:hex (30:70) to give pure product formula 3a as a yellow oil (2.14g, 68%). ¹H NMR (60 MHz, CDCl₃) δ 7.70 (2H, d), 7.03-7.09 (6H, m), 6.64 (2H, d), 5.42-5.46 (2H, m), 3.62-4.20 (4H, m), 3.87 (6H, s), 1.45-1.94 (12H, m), 1.47 (6H, s).

### Step 3: Di-cyclopropanation of compound with formula 3a to generate compound with formula 4a [1,3-bis(2-(3-methoxy-4-((tetrahydro-2H-pyran-2-yl)oxy)phenyl)cyclopropyl)-2,2-dimethylpropane-1,3-dione].

Dimethylsufoxide (6ml) was added to a mixture of trimethylsulfoxonium iodide (TMSOI ) (409mg, 1.86mmol) and sodium hydride (NaH) (74mg, 1.86mmol) and stirred at RT until evolution of hydrogen gas ceased and a clear solution was obtained (approximately 45min). This solution was added to a solution of (1E,6E)-1,7-bis(3-methoxy-4-((tetrahydro-2H-pyran-2-yl)oxy)phenyl)-4,4-dimethylhepta-1,6-diene-3,5-dione (500mg, 0.86mmol) in DMSO (2ml) and the mixture stirred at R.T. for 2h. At this time, TLC [(20uL aliquot into saturated NH₄Cl:EA (400uL:400uL); elution using EA:hex (30:70)] showed the formation of three major products and no S.M. The reaction mixture was diluted with EA (50ml) and washed with saturated NaCl (2 X 50ml), dried with Na₂SO₄, filtered and the solvents removed under reduced pressure to give a light yellow oil (514mg). The crude product was purified by FC on silica gel (50g) eluting with EA:hex (25:75) to give pure product formula 4a as a colorless oil (78mg, 15%). ¹H NMR (60 MHz, CDCl₃) δ 6.40-7.05 (6H, m), 5.24-5.30 (2H, m), 3.42-3.96 (4H, m), 3.79 (3H, s), 3.69 (3H, s), 1.09-2.43 (20H, m), 1.44 (6H, s).

### Step 4: Deprotection of the phenolic hydroxyl groups of compound with formula 4a to generate compound with formula 1 [1,3-bis(2-(4-hydroxy-3-methoxyphenyl)cyclopropyl)-2,2-dimethylpropane-1,3-dione]

Magnesium bromide etherate (MgBr₂*Et₂O) (91.5mg, 0.35mmol) was added to a solution of 1,3-bis(2-(3-methoxy-4-((tetrahydro-2H-pyran-2-yl)oxy)phenyl)cyclopropyl)-2,2-dimethylpropane-1,3-dione (70.0mg, 0.12mmol) in DCM (3.5ml) and stirred at R.T. for 25min. At this time, TLC [50uL aliquot into pH 5 buffer:EA (400uL:400uL); elution using EA:hex (40:60)] showed the clean formation of a major product. The reaction mixture was partitioned between pH 5 buffer (10ml) and DCM (5ml) and the layers separated. The organic layer was filtered through a plug of Na₂SO₄ and the solvents removed under reduced pressure to give a pale yellow oil (68mg). The crude product was purified by FC on silica gel (10g) eluting with EA:hex (40:60) to give pure product formula 1 as a pale yellow gel (48mg, 96% yield). HPLC-UV showed 93% purity. LC-MS (ESI⁻) showed expected mass [M-H]⁻ 423.5 (100%); ¹H NMR (60 MHz, CDCl₃) δ 6.37-6.89 (6H, m), 5.62 (2H, s), 3.85 (6H, s), 1.91-2.61 (4H, m), 1.18-1.70 (4H, m), 1.44 (6H, s).

### Example 8: UV-Vis spectra of compound with formula 1 in comparison to Curcumin.

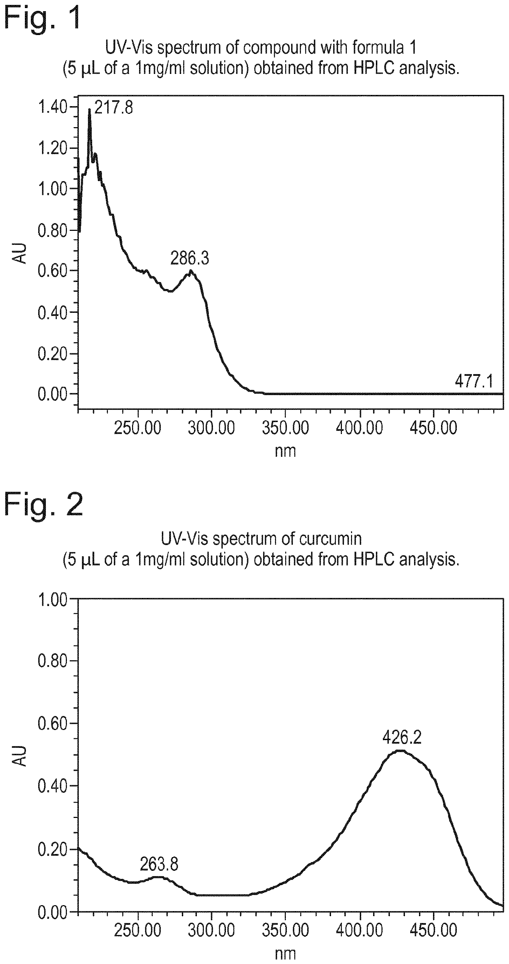

Figures 1 and 2 show the UV-Vis spectrum of compound with formula 1 and curcumin, respectively, obtained from HPLC analysis of 1mg/ml solution of each compound and using a photodiode array detector. Absorption in the range between 210-310nm for inventive compound with formula 1 is consistent with it's light/pale yellow color in its solid state and in solution. In contrast, strong absorption in the range between 360-480 nm for curcumin is consistent with the observed orange color of curcumin in it's solid state and in solution.

### Examples 9-11: Color assessment of a solution of compound with formula 1 in comparison to a solution of curcumin.

Solutions of compound with formula 1 and curcumin were prepared in ethyl acetate (200 µM) and the L*a*b* color space measured at room temperature (21 °C) using a Labscan XE instrument (Hunter Associates Labs Inc., Reston, VA) and processed with Universal Software (version 4.10). The results (Table 3) demonstrate that the color components a* and b* of compound with formula 1 (colorless) are largely reduced compared to curcumin (yellow) and more closely resemble those of the vehicle (ethyl acetate) which is colorless. These results are also consistent with the visual appearance of all solutions which show that in going from curcumin to compound with formula 1 the observed color is significantly reduced from yellow to colorless, the latter more closely resembling the color of the vehicle (colorless).

**Table 3**

| **Examples** | **Sample ^{a}** | **Appearance ^{b}** | **L*** | **a*** | **b*** |
|---|---|---|---|---|---|
| 9 | Vehicle (ethyl acetate) | colorless | 68.79 | -0.49 | 1.78 |
| 10 | Curcumin | yellow | 68.53 | -18.66 | 63.88 |
| 11 | Formula 1 | colorless | 68.62 | -0.68 | 2.49 |

| | | | | | |
|---|---|---|---|---|---|
| ^{a} Solutions prepared at 200 µM concentration; ^{b} Visual appearance of solution. | | | | | |

## Claims

1. A compound having the chemical structure of formula 1 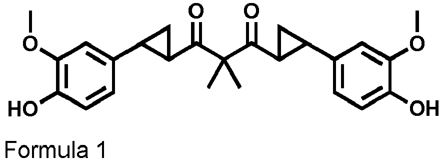 1,3-bis(2-(4-hydroxy-3-methoxyphenyl) cyclopropyl)-2,2-dimethylpropane-1,3-dione.

2. A personal care composition comprising (i) a compound as claimed in claim 1 and (ii) a cosmetically acceptable base.

3. A composition as claimed in claim 2 in the form of an oral care, or a skin, scalp or hair care product.

4. A composition as claimed in claim 3 in a form of a solid, soft solid, liquid, emulsion, microemulsion, lotion, cream, gel, or aerosol forms.

5. A process to prepare a compound of formula 1 as claimed in claim 1 comprising the steps:
(i) protection of the phenolic hydroxyl groups of curcumin with a suitable protecting group P to generate a compound with formula 2 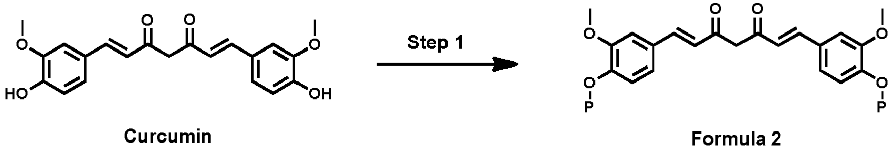
(ii) di-methylation of compound with formula 2 to generate a compound with formula 3 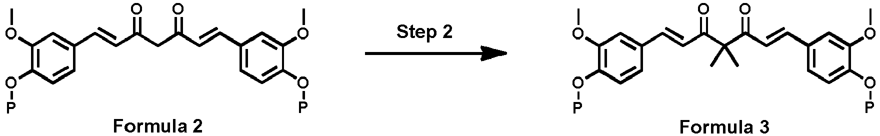
(iii) (di-cyclopropanation of compound with formula 3 to generate a compound with formula 4. 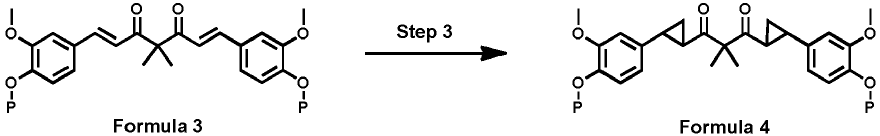
(iv) deprotection of the phenolic hydroxyl groups of compound with formula 4 to generate compound with formula 1.

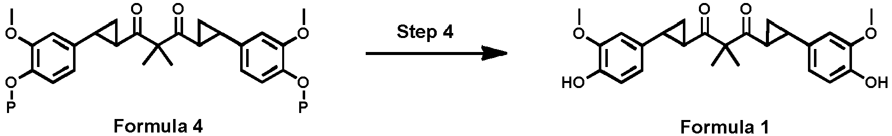

6. A compound as claimed in claim 1 for use as a medicament.

7. A compound as claimed in claim 1 for use in treating or reducing inflammation on a topical surface of a human or animal body.

8. A personal care composition as claimed in any one of the preceding claims 2 to 4 for use as a medicament.

9. A personal care composition as claimed in any of claims 2 to 4 for use in treating or reducing inflammation on a topical surface of a human or animal body.

## Patentansprüche

1. Verbindung der chemischen Struktur der Formel 1 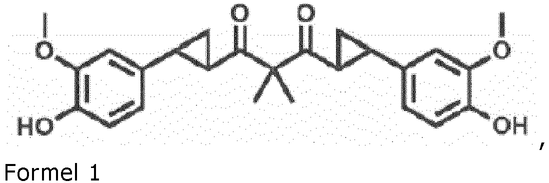 1,3-Bis(2-(4-hydroxy-3-methoxyphenyl)cyclopropyl)-2,2-dimethylpropan-1,3-dion.

2. Körperpflegezusammensetzung, umfassend (i) eine Verbindung, wie im Anspruch 1 beansprucht, und (ii) eine kosmetisch akzeptable Grundlage.

3. Zusammensetzung, wie im Anspruch 2 beansprucht, in Form eines Mundpflege- oder eines Haut-, Kopfhaut- oder Haarpflegeprodukts.

4. Zusammensetzung, wie im Anspruch 3 beansprucht, in Form eines Feststoffs, eines weichen Feststoffs, einer Flüssigkeit, einer Emulsion, einer Mikroemulsion, einer Lotion, einer Creme, eines Gels oder einer Aerosolform.

5. Verfahren zur Herstellung einer Verbindung der Formel 1, wie im Anspruch 1 beansprucht, umfassend die Schritte:
(i) Schützen der phenolischen Hydroxylgruppen von Curcumin mit einer geeigneten Schutzgruppe P, um eine Verbindung der Formel 2 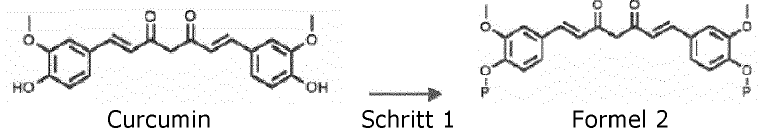 zu erzeugen;
(ii) Di-Methylierung der Verbindung der Formel 2, um eine Verbindung der Formel 3 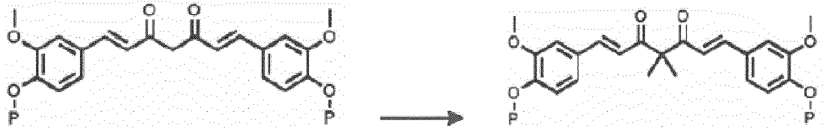 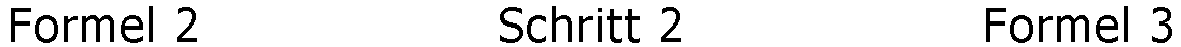 zu erzeugen;
(iii) (Di-Cyclopropanierung der Verbindung der Formel 3, um eine Verbindung der Formel 4 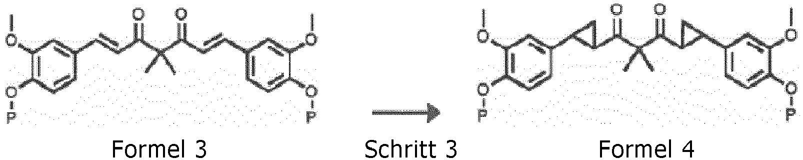 zu erzeugen;
(iv) Entschützen der phenolischen Hydroxylgruppen der Verbindung der Formel 4, um die Verbindung der Formel 1 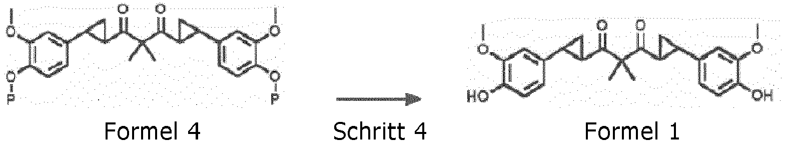 zu erzeugen.

6. Verbindung, wie im Anspruch1 beansprucht, zur Verwendung als Arzneimittel.

7. Verbindung, wie im Anspruch 1 beansprucht, zur Verwendung bei der Behandlung oder dem Vermindern von Entzündungen auf der topischen Oberfläche eines menschlichen oder tierischen Körpers.

8. Körperpflegezusammensetzung, wie in irgendeinem der vorhergehenden Ansprüche 2 bis 4 beansprucht, zur Verwendung als Arzneimittel.

9. Körperpflegezusammensetzung, wie in irgendeinem der Ansprüche 2 bis 4 beansprucht, zur Verwendung bei der Behandlung oder dem Vermindern von Entzündungen auf einer topischen Oberfläche eines menschlichen oder tierischen Körpers.

## Revendications

1. Composé ayant la structure chimique de formule 1 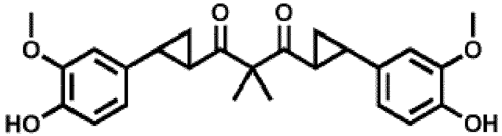 1,3-bis(2-(4-hydroxy-3-méthoxyphényl)cyclopropyl)-2,2-diméthylpropane-1,3-dione.

2. Composition pour l'hygiène personnelle comprenant (i) un composé selon la revendication 1 et (ii) une base cosmétiquement acceptable.

3. Composition selon la revendication 2 dans la forme d'un produit pour l'hygiène orale ou pour l'hygiène de la peau, du cuir chevelu ou des cheveux.

4. Composition selon la revendication 3 dans une forme d'un solide, des formes de solide mou, liquide, émulsion, microémulsion, lotion, crème, gel ou aérosol.

5. Procédé pour préparer un composé de la formule 1 selon la revendication 1 comprenant les étapes de :
(i) protection des groupes hydroxyle phénolique de curcumine avec un groupe protecteur approprié P pour produire un composé avec la formule 2 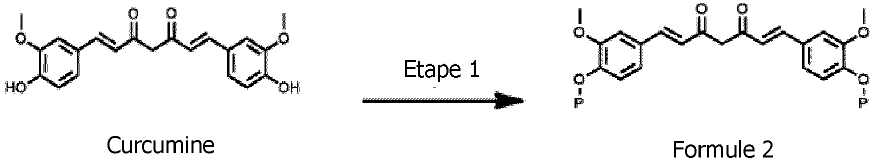
(ii) di-méthylation de composé avec la formule 2 pour produire un composé avec la formule 3 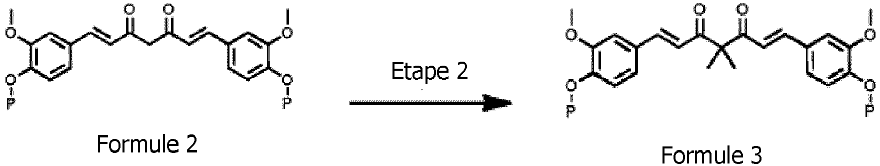
(iii) di-cyclopropanation de composé avec la formule 3 pour produire un composé avec la formule 4. 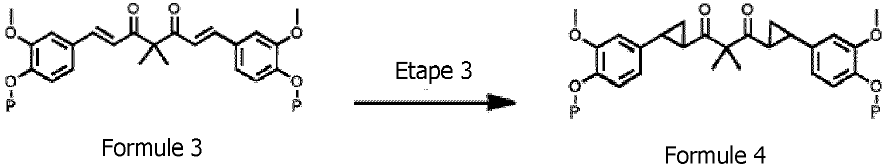
(iv) déprotection des groupes hydroxyle phénolique de composé avec la formule 4 pour produire un composé avec la formule 1.

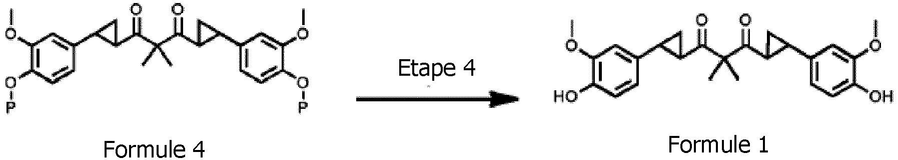

6. Composé selon la revendication 1 pour une utilisation comme un médicament.

7. Composé selon la revendication 1 pour une utilisation dans le traitement ou la réduction d'inflammation sur une surface topique d'un corps humain ou animal.

8. Composition pour l'hygiène personnelle selon l'une quelconque des revendications 2 à 4 précédentes pour une utilisation comme un médicament.

9. Composition pour l'hygiène personnelle selon l'une quelconque des revendications 2 à 4 pour une utilisation dans le traitement ou la réduction d'inflammation sur une surface topique d'un corps humain ou animal.
